(19) **Europäisches Patentamt**
**European Patent Office**
**Office européen des brevets**

(11) **EP 1 284 698 B1**

(12) **EUROPEAN PATENT SPECIFICATION**

(45) Date of publication and mention
of the grant of the patent:
**18.07.2007 Bulletin 2007/29**

(51) Int Cl.:
***A61F 9/00*** *(2006.01)*        ***A61F 2/16*** *(2006.01)*

(21) Application number: **01936400.9**

(22) Date of filing: **23.05.2001**

(86) International application number:
**PCT/EP2001/006040**

(87) International publication number:
**WO 2001/089435 (29.11.2001 Gazette 2001/48)**

(54) **METHOD OF PRE-SELECTING A POLYMERIZABLE FLUID FORMED INTO AN INTRAOCULAR LENS**

**VERFAHREN ZUR VORWAHL EINES POLYMERISIERBAREN FLUIDS ZUM EINSATZ IN EINE INTRAOKULARE LINSE**

**PROCEDE DE PRESELECTION DE FLUIDE POLYMERISABLE FA ONNE EN CRISTALLIN ARTIFICIEL**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **24.05.2000 SE 0001934**

(43) Date of publication of application:
**26.02.2003 Bulletin 2003/09**

(73) Proprietor: **AMO Groningen B.V.**
**9700 AX Groningen (NL)**

(72) Inventors:
• **TERWEE, Thomas, Henricus, Marie**
**NL-9301 PK Roden (NL)**

• **WEEBER, Hendrik, Albert**
**NL-9278 NL Groningen (NL)**
• **PIERS, Patricia, Ann**
**NL-9726 JK Groningen (NL)**

(74) Representative: **Holmberg, Martin Tor**
**Bergenstrahle & Lindvall AB**
**P.O. Box 17704**
**118 93 Stockholm (SE)**

(56) References cited:
**WO-A-00/22459            WO-A-99/47185**
**US-A- 4 542 542**

## Description

### Field of invention

The present invention refers to a method of pre-selecting a polymerizable fluid capable of being formed into an intraocular lens implant after being introduced into the capsular bag of the eye from which an impaired natural lens has been surgically removed.

### Background of invention

In the field of ophthalmic cataract surgery, wherein a defect natural lens is replaced with an artificial lens, there has been a development towards lenses and methods, which inflict less surgical trauma. For many years most of the IOLs were made of poly(methylmethacrylate) (PMMA), a material with good optical characteristics and compatibility with tissues in the eye. A disadvantage of PMMA, however, is that it is a very rigid material and a surgical incision must be made large enough, at least 5-6 mm, for the implantation of the lens. With improved devices for removal of the natural lens by phacoemulsification, requiring only a rather small incision, there was a need for lenses with deformable optics, as disclosed in the US Patent No. 4,573,998 (Mazzocco). There are presently several types of foldable intraocular lenses on the market which can be inserted through a considerably smaller incision of about 3 to 4 mm, for example made from specifically designed silicone materials.

Even with the mentioned types of improved implantable IOLs, now available on the market, there is still a desire to obtain a lens which admits the use of an even smaller incision and behaves like the natural lens in the eye, i.e. will be accommodating with a focal point regulated by action of the ciliary muscle in the eye. In order to allow for a really small incision it would be necessary to form the lens inside the eye from a solution which is injected into the capsular bag or into a balloon placed inside the bag by means of a standard injection needle.

IOLs formed from an injected solution of a silicone prepolymer, crosslinker and catalyst have already been suggested in US patents 5,278,258 and US 5,391,590 (Gerace et al). Generally low temperature curing at body temperature means a slow process and up to 12 hours may be needed to complete their setting and their slow setting may result in material leakage out of the capsular bag through the surgical incision. In order to overcome this problem, US Patents No. 4,542,542 and US 4,608,050 (Wright et al) disclose such a silicon based injected system which is partially cured by heat in the vicinity of the injection hole of the capsular bag to accomplish a first sealing effect.

Alternatively to thermocured silicone systems, photopolymerizable lens materials have been suggested which are activated after injection into the capsular bag by light in the presence of a photoinitiator. In the articles by Hettlich et al in German J Ophthalmol (1992), Vol. 1, pages 342-5 and 346-349, there are disclosures of how to employ photopolymerization of a monomer system injected into the capsular bag of the eye. An example of such an injectable system is also described in EP 414219, in which the liquid composition comprises a difunctional acrylate and /or methacrylate ester and a photoinitiator capable of being polymerized by light of a wavelength range between 400-500 nm. Further, the International patent application PCT/EP99/04715 is directed to an injectable photocurable aqueous solution of pre-polymerized units which is capable of forming a lens implant with a suitable elasticity modulus after final a crosslinking process triggered by visible light. Even if suitable polymerizable systems are at hand for preparing injectable lenses, there is still a considerable problem to obtain control of the refractive outcome of the eye after implantation. Accordingly, Hettlich et al suggested that by filling the capsular bag to varying degrees, or alternatively influencing the anterior or posterior capsular curvature, refractive control eventually would become possible.

O Nishi et al in Arch. Ophthalmol., 1997, Vol. 115, 507-510 describe experiments with direct injection of silicone material into the emptied capsular bag in cadaver pig eyes with subsequent plugging of the capsule and molding of the silicone into a synthetic lens. By using this procedure, the ability of the capsular bag to mold the injected silicone was investigated. It was found that different values of accommodation amplitude could be accomplished dependent on if the implanted lens was molded with a zonal tension applied on the capsule compared to when said tension was abolished. Nishi et al suggested that when (if) the eye is atropinized postoperatively, the lens capsule will conform to its non-accommodated state, which should yield the optimal amplitude of accommodation according to the investigated lens refilling principle.

Although there has been a considerable progress in the development of materials and surgical techniques for injectable lenses, considerable efforts are still needed to control the refractive outcome of this type of lenses. In particular, selection methods of suitable materials and improved control of the lens forming process after refilling the capsule will be necessary to carefully predict the refractive outcome of the eye subjected to lens replacement.

### Description of the invention

The present invention relates to a method of selecting suitable polymerizable fluid to be introduced into the capsular bag of the eye and subsequent polymerization into an intraocular lens.

More specifically, the present invention relates to a method of pre-selecting a polymerizable fluid to be introduced into the capsular bag and formed to an implanted replacement lens for an impaired natural lens. Typically, the natural lens to be replaced suffers from cataract formation, but also presbyopic lenses, i.e. lenses having completely or partially lost their capacity of accommodation, are also conceivable to be substitute within the context of the presently invented method. The polymerizable fluid is capable of being formed into an intraocular lens by means of one ore several polymerization reactions. The resulting lens is intended to provide the eye with a determined desired refractive outcome value estimated as optically suitable or ideal for the individual elected to undergo surgery. The pre-selection method comprises measuring of selected eye dimensions including corneal curvature (anterior or posterior curves or both), the axial length of the eye and the anterior chamber depth. The person skilled in this technology is knowledgeable of several measurement methods to obtain this information and thereby finding relevant information about the eye, including the refractive value of the cornea. From these values the shape and the volume of capsular bag is estimated and thereby it is possible to calculate the quantity and refractive index of the polymerizable fluid to be introduced in the capsular bag in order to obtain a refractive value that sufficiently complies with the desired refractive outcome value of the individual eye. Typically, such calculations comprise the determination of a lens model refractive value (the refractive value necessary for the lens to restore the vision) from the measured corneal refractive value and the desired refractive outcome value. By further estimating the shape and thereby the volume of the individual capsular bag together with lens model refractive value, a quantity of polymerizable fluid with a specific refractive index is determined. According to one embodiment of the method, a polymerizable fluid then is selected from a kit of polymerizable fluids with a range of different refractive indices. In practical terms, the surgeon can select the determined quantity of the fluid of the kit having a refractive index value, which is most compatible to the estimated value of the refractive outcome value. Alternatively, a polymerizable fluid having an identical refractive index value to what has been estimated can be ordered from a manufacturer. Preferably, such kits of polymerizable fluids will have a range of refractive indices varying from about 1.39 to about 1.6. As discussed in more detail below, several suitable polymerizable fluids are conceivable for the kit which comply with such requirements as being easy to inject with conventional equipment, having suitably high specific gravity and providing options to obtain suitably high refractive indices and yet obtaining desirable mechanical characteristic for intraocular lenses after polymerization, including sufficiently low modulus to obtain an implanted lens, that can undergo accommodation when influenced by the ciliary muscles of the eye. The kit can typically comprise a range of such fluids filled in multi-compartment containers, separately stored from agents necessary to bring about the polymerization. Advantageously, the multi-compartment containers are provided with means to establish fluid communication between the containers just prior to the administration into the capsular bag and with means, either to inject the fluid, or to operate on the container with a conventional injection device. Many such containers are known to the skilled person and will not be described herein in more detaiL

It is preferable that the polymerizable fluid shall be introduced in the capsular bag by means of injection, suitably through the orifice already created in the wall during the removal of the impaired natural lens. For this purpose, it is a prerequisite that the polymerizable fluid has a sufficiently low viscosity so it can be efficiently injected through a standard cannula with an 18 Gauge needle or finer. Preferably, the polymerizable fluid has a viscosity below about 60000 cSt and more preferably, below about 8000 cSt.

According to one aspect, the polymerizable fluid which is used in the method of the present invention comprises a polymerizable polysiloxane composition, suitably also comprising a crosslinking agent to participate in the polymerizing forming process, i.e. a crosslinking process. According to one alternative of this aspect, the polysiloxane composition further comprises a catalyst activated by heat to initiate the crosslinking process. In another alternative, the polysiloxane composition comprises a photoinitiator that suitably is activated by visible light, in particular blue light. A useful polysiloxane composition can be found in the International Patent Application PCT/EP99/07780 that describes silicone compositions adapted for being thermocured in the capsular bag. The polymerization after injection such a composition can be initiated by raising the temperature of the rinsing fluid to a value necessary to activate the catalyst driven polymerization. Typically, such a increase in temperature can be from about 20 to about 40°C. Alternatively, it is conceivable to use the photocurable compositions designed for intraocular lens production directly in the capsular bag of the eye, as described in the International Patent Application PCT/EP99/04715. From the teachings of these documents, the skilled person can readily obtain a wide range of polymerizable polysiloxane compositions suitable for injection into the capsular bag, having a range of different refractive indices varying from about 1.39 up to 1.6, as is suitable for the above-mentioned kit for selecting an appropriate polymerizable fluid. Both these documents, which herewith are incorporated as references in their entirety, provide polysiloxanes designed for injection into the capsular bag which by a specific selection of substituents on the polysiloxane backbone enables suitable variation range in refractive index, while still retaining characteristics of sufficiently high density (preferably higher than about 1.0 $g/cm^3$) and excellent mechanical characteristics for lens production.

According to an alternative aspect, the polymerizable fluid comprises an aqueous composition of a hydrophilic polymer carrying sites for crosslinking, wherein said aqueous composition further comprises a crosslinker. Preferably, the formation of a lens implant is initiated by activating a photoinitiator by irradiation of a predetermined wavelength or range of wavelengths. Most suitable in the context of the present method is to select a photoinitiator activated by visible light, preferably blue light. Examples of such compositions are found in the International Patent Application published as WO 99/47185, wherein crosslinkable hydrophilic units of different polymers are disclosed.

Consequently the present invention admits that the patient is provided with an intraocular lens with an accurately determined refractive value that can restore the vision to a predetermined value. This is a considerable advantage when compared to conventional surgery with stiff or foldable lenses no refractive adjustments can be performed once the lens is inserted into the capsular bag. In combination with the fact that the present invention permits a surgical intervention causing less trauma from large incisions in the eye, it is obvious that highly advantageous contributions to the art are provided.

## Detailed and exemplifying description of the invention

The inventive method is a directed to securing that the intended post-operative refraction is achieved by predicting the amount of fluid and the refractive index of the fluid pre-operatively, which guarantee a correct post-op refraction. It is also possible to determine the appropriate volume and refractive index of the polymerizable fluid prior to the surgical procedure, then during the operation, the refraction is further fine-tuned to the intended value. When the human lens is impaired, for instance by cataract or presbyopia, the impaired lens can be removed out of the lens capsule. Thereafter a polymerizable fluid can be injected into the capsule and polymerized. The new lens is molded by the capsule. The newly molded lens must have the correct lens power in order to give the patient the intended postoperative refraction. The lens power depends largely on the amount of injected fluid and the refractive index of the fluid. This means that the lens power can be controlled by these two parameters. Based on the predictions of the volume and refractive index for a specific patient, the surgeon can be provided a kit of materials at the operating table, from which he select the right one for the specific patient.

In the following example, a method is described which predicts the volume and refractive index of the polymerizable fluid, based on measurements on the individual patients and combined with data for the average human eye.

### Example 1

The determination is based on the combination of two sets of data:

    1. General data of the human eye, measured on a representative population
    2. Measurements of the individual patient

1. General data

*Lens thickness*

The lens thickness is very much depending on age. Within an age group, the spread in lens thickness is very small. Shum, Ko, Ng and Lin (1993) measured the lens thickness of a group of 76 subjects of virtually the same age (s.d. 1.2 month). On an average lens thickness of 3.49 mm he found a standard deviation of 0.02 mm, which is 0.5%. The age relation of the lens thickness is best described by Koretz, Kaufman, Neider and Goeckner (1989), who found a relation of

$$LT = 3.220 + 0.021 * A \tag{1}$$

    LT = lens thickness [mm]
    A = age [y]

When using this relation, the actual input for the calculation is age, and not lens thickness.

*Relation between anterior and posterior lens radius*

The actual lens radii of an individual patient may differ a lot, however there is always a certain relation between the two. Based on measurements on human cadaver eyes, Glasser & Campbell (1999) found the relation of:

$$Rp = -0.261*Ra - 2.631 \tag{2}$$

Rp = posterior radius [mm]
Ra = anterior radius [mm]

*Relation between lens equatorial diameter and lens focal length*

The lens equatorial diameter is hidden behind the iris. It can not be measured with the equipment that is normally available in the ophthalmic practice. Therefore a reasonable estimate can be made, using the relation that was found by Glasser & Campbell (1999), based on measurements on human cadaver eyes:

$$LD = 0.0502*FL + 5.288 \tag{3}$$

LD = lens equatorial diameter
FL = lens focal length

*Relation between the natural lens focal length and the refilled lens focal length*

The lens capsule and the lens do not need to have the same shape. As a result it is possible that after refilling the lens, the shape of the lens has changed. This was seen in calculations of lens refilling. This phenomenon also follows from the results of Glasser & Campbell (1999): For most lenses the focal length changes after decapsulation of the lens. However, this effect disappears at the age of 60 years, which corresponds to the age of full presbyopia. From this it can be concluded that the focal length of the refilled lens will be equal to the focal length of the original lens, provided that the refill material has the refractive index of natural lens material.

Measurements on the individual patient

*Keratometer*

With the keratometer, the corneal power is measured. This is currently a standard measurement in cataract surgery. Alternatively, the corneal curvature (radius) can be measured. The relation between corneal curvature and corneal power is:

$$K = 337.5/Rc \tag{4}$$

K = Corneal power [D]
Rc = Curvature radius of the cornea

*A-scan*

With an A-scan, the axial dimensions of the eye can be measured. Also this is currently standard practice in cataract surgery. In general, it results in a measure of the anterior chamber depth and the total axial length of the eye.

*Refraction and refraction history*

The refraction is measured by the optometrist. When the patient is currently blind, the refraction can not be measured. In such a case there are two alternatives:

1. The refraction history of the patient's eye, during the period that the patient was not blind.

2. The refraction and/or the refraction history of the patient's fellow eye.

Calculation scheme

1. Determination of the lens thickness, from the age of the patient
2. Determining the radii of the lens, based on the known optical surfaces and refraction of the eye.
3. Determining the focal length of the natural lens
4. Determining the lens equatorial diameter, based on the lens focal length
5. Determining the volume of the natural lens.
6. Based on the desired post-op refractive outcome, select the appropriate refractive index.

The volume to be used is equal to the volume of the natural lens. The refractive index of the material is adapted, so that the predetermined refractive outcome for the patient will be reached.

Patient data:

Age: 63 year

| | | |
|---|---|---|
| Results of the ophthalmic exam: | | |
| Keratometer reading: | 43.7 | Diopter |
| A-scan:Axial length | 23.35 | mm |
| Anterior chamber depth | 3.25 mm | |
| Historic refraction: spherical equivalent (stable). | +2.5 | Diopter |

Accordingly, the calculations according to the calculation scheme is :

1. According equation (1), the lens thickness is 4.543 mm.
2. According equation (4), the cornea has a radius of 7.723 mm.

The length of the vitreous is the axial length, minus the anterior chamber depth and minus the lens thickness. So far the optical system is:

| Surface | Name | Radius | Thickness | Refractive index |
|---|---|---|---|---|
| 1 | Cornea | +7.723 | 3.25 | 1.3375 |
| 2 | Lens | Ra | 4.543 | 1.422 |
| 3 | Vitreous | Rp | 15.557 | 1.336 |
| 4 | Retina | - | - | - |

Since, according equation (2), Rp is a function of Ra, there is only one variable in this system. This variable can be solved, using the condition that it has to result in the known or historic refraction. Here a paraxial ray tracing procedure is used, adapted from the spreadsheet that is used to calculate A-constants for IOL's. This results in the lens radii: (Refraction Rx = spectacle refraction. For modeling the eye, the spectacle is made of Crown glass (Agarwal's principles of Optics and Refraction), 2 mm thick, with it's anterior surface 14mm in front of the cornea).

Ra = 12.286 mm
Rp = -5.838

3. The focal length of the natural lens is determined by the dimensions and refractive index:

Ra = 12.286 mm
Rp = -5.838 mm
Thickness = 4.543
Refractive index = 1.422
⇨ Lens power (P), according the thick lens equation is 21.40 diopter and the focal length is 1336/P = 62.425 mm.

*4.* Equatorial diameter, according equation (3) is 8.422 mm.

*5.* The volume of the lens, based on an ellipsoid, with the known thickness and equatorial diameter is 186.7 mm³.

The volume of an ellipsoid is: $V = \frac{4}{3} * \pi * a^2 * b$

With:

    a = diameter/2

    b = thickness/2

*6.* The refractive index can now be chosen for a specific post-operative refractive outcome. An index of 1.422 will result in the pre-op (historic) refraction of 2.5 diopter. The result of different refractive indices can be calculated by paraxial ray tracing, and results in the following table (Rx = post-op refraction):

| Rx | η |
| --- | --- |
| -3 | 1.457 |
| -2 | 1.451 |
| -1 | 1.445 |
| 0 | 1.439 |
| 1 | 1.432 |
| 2 | 1.426 |
| *2.5* | *1.422* |
| 3 | 1.418 |
| 4 | 1.411 |
| 5 | 1.403 |
| 6 | 1.395 |

References

Agarwal, L.P. (1998). Agarwal's Principles of Optics and Refraction (5 ed.). New Dehli: CBS Publishers & Distributers.

Glasser, A., & Campbell, M.C.W. (1999). Biometric, optical and physical changes in the isolated human crystalline lens with age in relation to presbyopia. Vision Research, 39(11), 1991-2015.

Koretz, J.F., Kaufman, P.L., Neider, M.W., & Goeckner, P.A. (1989). Accommodation and presbyopia in the human eye--aging of the anterior segment. Vision Res, 29(12), 1685-1692.

Shum, P.J., Ko, L.S., Ng, C.L., & Lin, S.L. (1993). A biometric study of ocular changes during accommodation (see comments). Am J Ophthalmol, 115(1), 76-81.

**Claims**

1. A method of pre-selecting a polymerizable fluid capable of being formed into an intraocular lens implant after being introduced into the capsular bag of the eye from which an impaired natural lens has been surgically removed, comprising the steps of:

    (a) determining a desired refractive outcome value suitable for the eye;

    (b) measuring one or several eye dimensions selected from corneal curvature, axial length of the eye or anterior chamber depth;

    (c) estimating the shape of the capsular bag;

(d) calculating the quantity and the refractive index of the polymerizable fluid to be introduced in the capsular bag for obtaining a refractive value that sufficiently complies with the desired refractive outcome of (a);

2. A method according to claim 1, comprising determining a lens model refractive value from a corneal refractive value obtained from step (b) and the desired refractive outcome value (a).

3. A method according to claim 2, comprising obtaining the quantity and refractive index of the polymerizable fluid from the lens model refractive value and the estimations in step (c).

4. A method according to claim 1, comprising selecting a polymerizable fluid, having the most compatible value to that obtained in step (d), from a kit of polymerizable fluids having a range of refractive indices.

5. A method according to claim 4, wherein said kit has a range fluids having refractive indices varying from about 1.41 to about 1.6.

6. A method according to claim 5, wherein said kit comprises a range of fluids filled in multi-compartment containers separately stored from agents necessary to bring about the polymerization.

7. A method according to claim 6, wherein fluid communication can be established between said compartments just prior to the fluid administration to the capsular bag.

**Patentansprüche**

1. Verfahren zum Vorauswählen eines polymerisierbaren Fluids, welches eingerichtet ist, die Form eines intraokularen Linsen-Implantats anzunehmen, nachdem es in die Kapseltasche des Auges, aus welcher eine geschädigte natürliche Linse operativ entfernt wurde, eingebracht wurde, aufweisend die folgenden Schritte:

(a) Ermitteln eines gewünschten, für das Auge geeigneten Brechungs-Ergebnis-Wertes;
(b) Messen einer einzelnen oder mehrerer Augen-Dimensionen ausgewählt von Hornhaut-Krümmung, axiale Länge des Auges oder Vorderkammer-Tiefe;
(c) Abschätzen der Form der Kapseltasche;
(d) Berechnen der Menge und des Brechungsindex des in die Kapseltasche einzubringenden polymerisierbaren Fluids zum Erzielen eines Brechungsindex, der das gewünschte Brechungs-Ergebnis von (a) ausreichend erfüllt.

2. Verfahren gemäß Anspruch 1, aufweisend Ermitteln eines Linsenmodell-Brechungswertes aus einem in Schritt (b) erzielten Hornhaut-Brechungswert und dem gewünschten Brechungs-Ergebnis-Wert (a).

3. Verfahren gemäß Anspruch 2, aufweisend Erzielen der Menge und des Brechungsindex des polymerisierbaren Fluids aus dem Linsenmodell-Brechungswert und den Abschätzungen in Schritt (c).

4. Verfahren gemäß Anspruch 1, aufweisend Auswählen eines polymerisierbaren Fluids, das den kompatibelsten Wert zu dem in Schritt (d) erzielten aufweist, aus einem Satz von polymerisierbaren Fluids, die einen Bereich an Brechungsindizes aufweisen.

5. Verfahren gemäß Anspruch 4, wobei der Satz einen Bereich an Fluids mit Brechungsindizes aufweist, die von ungefähr 1,41 bis ungefähr 1,6 variieren.

6. Verfahren gemäß Anspruch 5, wobei der Satz einen Bereich an in Mehr-Abteil-Behältern eingefüllten Fluids aufweist, die getrennt von zum Bewirken der Polymerisation notwendigen Wirkstoffen aufbewahrt werden.

7. Verfahren gemäß Anspruch 6, wobei Fluid-Übertragung zwischen den Abteilen kurz vor der Fluid-Verabreichung in die Kapseltasche eingerichtet werden kann.

**Revendications**

1. Procédé de présélection d'un fluide polymérisable capable d'être formé dans un implant de lentille intraoculaire après avoir été introduit dans le sac capsulaire de l'oeil duquel une lentille naturelle détériorée a été enlevée chirurgicalement, comprenant les étapes consistant à :

(a) déterminer une valeur de sortie de réfraction souhaitée convenable pour l'oeil ;
(b) mesurer une ou plusieurs dimensions de l'oeil choisies parmi la courbure de la cornée, la longueur axiale de l'oeil ou la profondeur de la chambre antérieure ;
(c) estimer la forme du sac capsulaire ;
(d) calculer la quantité et l'indice de réfraction du fluide polymérisable destiné à être introduit dans le sac capsulaire pour obtenir une valeur de réfraction qui satisfait suffisamment à la sortie de réfraction souhaitée de (a) .

2. Procédé selon la revendication 1, comprenant la détermination d'une valeur de réfraction du modèle de lentille à partir d'une valeur de réfraction de la cornée obtenue à l'étape (b) et de la valeur de sortie de réfraction souhaitée (a).

3. Procédé selon la revendication 2, comprenant l'obtention de la quantité et de l'indice de réfraction du fluide polymérisable à partir de la valeur de réfraction du modèle de lentille et des estimations de l'étape (c).

4. Procédé selon la revendication 1, comprenant la sélection d'un fluide polymérisable, ayant la valeur la plus compatible à celle obtenue à l'étape (d), à partir d'une trousse de fluides polymérisables ayant une plage d'indices de réfraction.

5. Procédé selon la revendication 4, dans lequel ladite trousse comprend des fluides dans une plage ayant des indices de réfraction variant d'environ 1,41 à environ 1,6.

6. Procédé selon la revendication 5, dans lequel ladite trousse comprend une plage de fluides remplis dans des réservoirs à plusieurs compartiments conservés séparément des agents nécessaires pour provoquer la polymérisation.

7. Procédé selon la revendication 6, dans lequel ladite communication fluidique peut être établie entre lesdits compartiments juste avant l'administration du fluide au sac capsulaire.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4573998 A, Mazzocco
- US 5278258 A
- US 5391590 A, Gerace
- US 4542542 A
- US 4608050 A, Wright
- EP 414219 A
- EP 9904715 W
- EP 9907780 W
- WO 9947185 A

**Non-patent literature cited in the description**

- **HETTLICH et al.** *German J Ophthalmol,* 1992, vol. 1, 342-5346-349
- **O NISHI et al.** *Arch. Ophthalmol.,* 1997, vol. 115, 507-510
- **AGARWAL, L.P.** *Agarwal's Principles of Optics and Refraction,* 1998
- **GLASSER, A. ; CAMPBELL, M.C.W.** Biometric, optical and physical changes in the isolated human crystalline lens with age in relation to presbyopia. *Vision Research,* 1999, vol. 39 (11), 1991-2015
- **KORETZ, J.F. ; KAUFMAN, P.L. ; NEIDER, M.W. ; GOECKNER, P.A.** Accommodation and presbyopia in the human eye--aging of the anterior segment. *Vision Res,* 1989, vol. 29 (12), 1685-1692
- **SHUM, P.J. ; KO, L.S. ; NG, C.L. ; LIN, S.L.** A biometric study of ocular changes during accommodation. *Am J Ophthalmol,* 1993, vol. 115 (1), 76-81